# EUROPEAN PATENT APPLICATION

(11) **EP 1 582 146 A2**
(43) Date of publication of application: **05.10.2005**
(21) Application number: 05006103.5
(22) Date of filing: 21.03.2005
(51) Int. Cl.: A61B 5/021

(54) **Vital sign processing apparatus and method**

(30) Priority: 26.03.2004 JP 2004093582
(71) Applicant: MATSUSHITA ELECTRIC INDUSTRIAL CO., LTD., Kadoma-shi, Osaka 571-8501 (JP)
(72) Inventor: Kenjou, Noriko, Osaka-shi Osaka 532-0003 (JP); Kawamura, Tatsurou, Kyotanabe-shi Kyoto 610-0351 (JP); Kamei, Akihito, Yawata-shi Kyoto 614-8295 (JP)
(74) Representative: Pautex Schneider, Nicole Véronique

(57) **Abstract**

The vital sign processing apparatus in the present invention includes: a vital sign measurement unit that measures a vital sign of a subject; a designated condition notification unit that notifies the subject of a designated condition to be followed during measurement of the vital sign using the vital sign measurement unit; a measurement condition recognition unit that recognizes, without input from the subject, a measurement condition at a time the vital sign is measured by the subject using the vital sign measurement unit; a condition comparison unit that compares the designated condition notified by the designated condition notification unit, with the measurement condition recognized by the measurement condition recognition unit; and a presentation unit that presents a comparison result from the condition comparison unit.

## Description

### BACKGROUND OF THE INVENTION

### (1) Field of the Invention

The present invention relates to a vital sign processing apparatus, and a vital sign processing system, for acquiring data in which a vital sign of a subject and measurement conditions at a time the vital sign is measured are associated with each other.

### (2) Description of the Related Art

As a conventional data acquisition system, there exists a system where, through the transmission of measurement data by a subject measuring his own vital signs (hereinafter referred to simply as subject), an acquisition apparatus acquires such data, and the acquired data is managed and processed (see Japanese Laid-Open Patent Application No. 2002-291069 Publication, page 8, FIG. 1, for example).

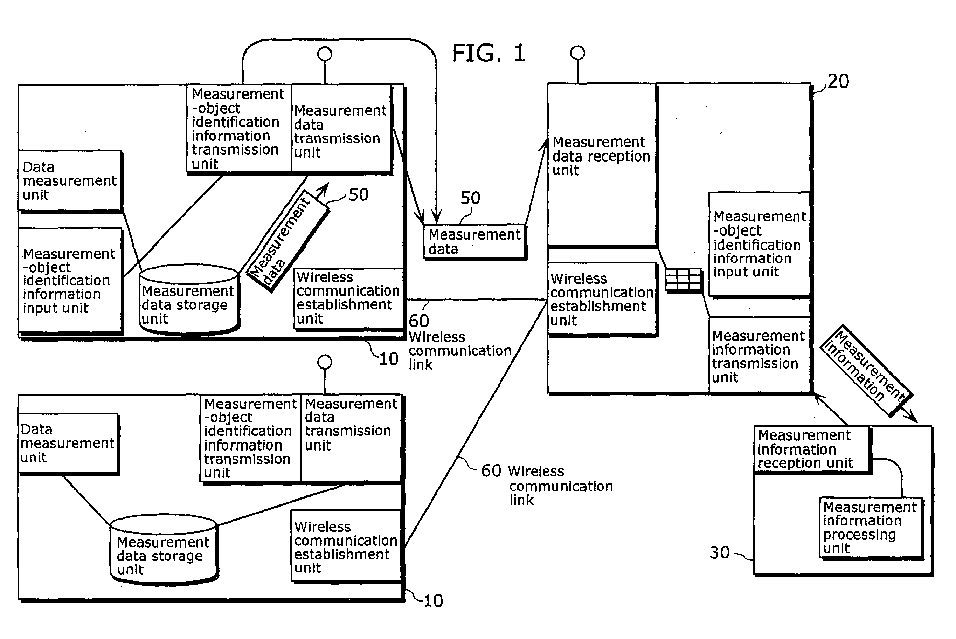

FIG. 1 is a diagram showing the conventional data acquisition system and data acquisition method described in the Japanese Laid-Open Patent Application No. 2002-291069 Publication.

In FIG. 1, a data-measuring subject measures measurement data 50, which is a vital sign to be measured, using a data measurement apparatus 10, and directly transmits the measurement data 50 to a data acquisition apparatus 20 via a wireless communication link 60. A data processing apparatus 30 performs processing corresponding to the measurement data received from the data acquisition apparatus 20.

To begin with, measurement data, which is the measured vital sign, reflects measurement conditions such as atmospheric temperature and humidity, and only when these measurement conditions are included, is it considered as having value as data. Accordingly, when data of a vital sign which include such measurement conditions can be acquired and accumulated, a useful database can be constructed.

However, in the conventional system for acquiring data, the acquisition apparatus is limited to managing, on a per-subject basis, the measurement data measured by a subject, and the selection and determination of the measurement conditions is largely left to the discretion of the subject. As such, in the case where the subject does not understand the meaning and significance in measuring vital signs, and the case where measured data is not understood, many instances arise where measurement is performed under unsuitable measurement conditions such as when vital signs are measured under measurement conditions based only on the judgment made by the subject, without regard to the intentions of the side acquiring the data.

In the conventional vital sign data acquisition system under the aforementioned circumstances, measurement data having low reliability and measurement conditions that are far from the desired conditions are included within the acquired measurement data. Accordingly, information created by analyzing a database constructed based on such unreliable measurement data may not necessarily be useful.

In this manner, in the acquisition of measurement data for constructing a database, it is very difficult to properly acquire, based on fixed measurement conditions, vital signs whose measurement initiatives and determination is dependent on the subject.

In addition, the subject is required to pay fees for the vital sign measurement per se and for an analysis result based on the vital signs, which makes wider measurement data acquisition difficult.

The present invention is conceived to solve such existing problems, and has as a first objective to provide a vital sign processing apparatus which enables extensive acquisition of more useful and highly reliable vital signs, and contributes to the construction, based on the acquired vital signs, of a database that can better contribute to health management.

Furthermore, it is the second objective of the present invention to provide a vital sign processing system which, while acquiring useful vital signs, further reduces financial costs arising from the obtainment of the subject's vital signs and analysis result thereof.

### SUMMARY OF THE INVENTION

In order to solve the existing problems, the vital sign processing apparatus in the present invention includes: a vital sign measurement unit operable to measure a vital sign of a subject; a designated condition notification unit operable to notify the subject of a designated condition for measuring the vital sign using said vital sign measurement unit; a measurement condition recognition unit operable to recognize, without input from the subject, a measurement condition at a time the vital sign is measured by the subject using said vital sign measurement unit; a condition comparison unit operable to compare the designated condition notified by said designated condition notification unit with the measurement condition recognized by said measurement condition recognition unit; and a presentation unit operable to present a result of the comparison made by said condition comparison unit.

According to this structure, it is possible to notify, as a designated condition, the measurement condition that complies with the type of the vital sign and the purpose of the processing result thereof, to the subject. Accordingly, the subject is able to know the designated condition in advance and gets a better understanding regarding the measurement, in addition, the preparations related to the measurement are made easier, and it becomes easier to have the measurement performed according to the designated condition.

Furthermore, by comparing the measurement condition at the time of the measurement using the vital sign measurement unit, with the designated condition, and presenting the result thereof, the subject is able to recognize whether or not the vital sign is measured according to the designated condition.

Accordingly, as the subject recognizes the importance of the designated condition and the willingness of the subject to accurately measure the vital sign in accordance with the designated condition, an improvement in the reliability of the vital sign measured by the subject is brought about.

The vital information processing apparatus may further include a compliance level creation unit operable to create a compliance level for the designated condition, based on the result of the comparison made by said condition comparison unit, wherein said presentation unit may be operable to present the compliance level created by said compliance level creation unit.

According to the present structure, it becomes possible, not just to indicate the result of the comparison between the designated condition and the measurement condition, but to specifically present whether or not the vital sign is measured according to the designated condition, and it becomes easier to recognize and verify whether or not an accurate measurement, which yields information that is useful to the subject, is achieved. Furthermore, by quantifying the compliance level, the compliance level becomes a guide to the assessment of reliability.

The vital sign processing apparatus may further include an additional information input unit through which additional information is inputted, the additional information being information that cannot be recognized by said measurement condition recognition unit, wherein said condition comparison unit may be operable to compare the designated condition with the additional information.

According to the present structure, as it is possible to obtain additional information that cannot be recognized by the vital sign processing apparatus, such as "how many hours after breakfast the vital sign measured" and "body feels languid today", compare such additional information with the designated information, and present the result of the comparison and the compliance level, the subject can be made to further recognize the meaning of the vital sign measurement, and verify this at the time of the measurement.

The vital sign processing apparatus may further include: an information accumulation unit operable to store the vital sign measured using said vital sign measurement unit, the measurement condition created by said measurement condition recognition unit, and the additional information inputted through said additional information input unit, in association with each other; and an accumulation judgment unit operable to judge, based on the compliance level, whether or not to accumulate the vital sign, the measurement condition, and the additional information, in said information accumulation unit.

According to the present structure, by accumulating together with the measurement condition and added information, only the vital sign measured in accordance with the designated condition, it becomes possible to accurately ascertain the physical condition and medical history of the subject, from this accumulated data. Accordingly, accumulation of useful and highly reliable vital signs is made possible, which is conducive to the construction of a database that can better contribute to health management.

The vital sign processing apparatus may further include a re-measurement information creation unit operable to judge whether or not re-measuring is necessary, based on the compliance level, wherein, in the case where said re-measurement information creation unit judges that re-measuring is necessary, and creates re-measurement information, said presentation unit may be operable to present the re-measurement information.

According to the present structure, accurate vital signs complying with the designated condition can be easily obtained.

The vital sign processing apparatus may further include a feedback information creation unit operable to create, based on the compliance level, feedback information for increasing motivation of the subject to take a measurement according to the designated condition, wherein said presentation unit may be operable to present the feedback information.

According to the present structure, by making the subject expect feedback information such as points that are convertible to cash, as a reward, it becomes possible to increase the motivation of the subject to measure vital signs more accurately, according to the designated condition.

In order to solve the existing problems, the vital sign processing system in the present invention includes a vital sign measurement apparatus that obtains a vital sign from a subject, and a vital sign acquisition apparatus that acquires the vital sign from said vital sign measurement apparatus, wherein said vital sign acquisition apparatus includes: a designated condition creation unit operable to create a designated condition for measuring the vital sign using said vital sign measurement apparatus; an acquisition apparatus-side output unit operable to output the designated condition, and said vital sign measurement apparatus includes: a measurement apparatus-side input unit operable to receive the designated condition; a designated condition notification unit operable to notify the subject of the designated condition created by said designated condition creation unit; a vital sign measurement unit operable to measure the vital sign of the subject; a measurement condition recognition unit operable to recognize, without input from the subject, a measurement condition at a time the vital sign is measured by the subject using said vital sign measurement unit; and a measurement apparatus-side output unit operable to output the vital sign and the measurement condition, said vital sign acquisition apparatus further includes: an acquisition apparatus-side input unit operable to receive the vital sign and the measurement condition; a condition comparison unit operable to compare the designated condition with the measurement condition, and said acquisition apparatus-side output unit is operable to output a result of the comparison made by said condition comparison unit.

According to the present structure, it is possible for the vital sign acquisition apparatus to create a designated condition and notify the designated condition to the subject. By notifying the designated condition to the subject in this manner, the subject is able to know the designated condition in advance and gets a better understanding regarding the measurement, in addition, the preparations related to the measurement are made easier, and it becomes easier to have the measurement performed according to the designated condition created by the vital sign acquisition apparatus.

Furthermore, by comparing the measurement condition during the actual measurement using the vital information measurement unit, with the designated condition, and outputting the result of the comparison, the subject is able to recognize whether or not the vital sign is measured according to the designated condition. Accordingly, as the subject recognizes the importance of the designated condition and the willingness of the subject to accurately measure the vital sign in accordance with the designated condition, an improvement in the reliability of the vital sign measured by the subject is brought about.

The vital sign acquisition apparatus may further include a compliance level creation unit operable to create a compliance level for the designated condition, based on the result of the comparison made by said condition comparison unit, and said acquisition apparatus-side output unit may be operable to output the compliance level created by the compliance level creation unit.

According to the present structure, it becomes possible, not just to indicate the result of the comparison between the designated condition and the measurement condition, but to specifically present whether or not the vital sign is measured according to the designated condition, and it becomes easier to recognize and verify whether or not an accurate measurement, which yields information that is useful to the subject, is achieved. Furthermore, by quantifying the compliance level, the compliance level becomes a guide to the assessment of reliability.

The vital sign acquisition apparatus may further include an information accumulation unit operable to store the vital sign and the measurement condition in association with each other; and an accumulation judgment unit operable to judge, based on the compliance level, whether or not to accumulate the vital sign and the measurement condition, in said information accumulation unit.

According to the present structure, it is possible to accumulate only a vital sign having a high compliance level with respect to the designated condition, and highly useful information can be obtained. Accordingly, accumulation of useful and highly reliable vital signs is made possible, which is conducive to the construction of a database that can better contribute to health management.

The vital sign acquisition apparatus may acquire the vital sign from the vital sign measurement apparatus, with a communication network serving as an intermediary. Furthermore, the vital sign acquisition apparatus may acquire the vital sign from the vital sign measurement apparatus, with a portable storage medium serving as an intermediary.

The vital sign measurement apparatus may further include an additional information input unit through which additional information is inputted, the additional information being information that cannot be recognized by said measurement condition recognition unit, said measurement apparatus-side output unit may be operable to further output the additional information, said acquisition apparatus-side input unit may be operable to further receive the additional information, and said condition comparison unit may be operable to further compare the designated condition with the additional information.

The vital sign acquisition apparatus may further include a feedback information creation unit operable to create, based on the compliance level, feedback information for increasing motivation of the subject to take a measurement according to the designated condition, and said acquisition apparatus-side output unit may be operable to output the feedback information.

In order to solve the existing problems, the vital information acquisition apparatus in the present invention is a vital sign acquisition apparatus that acquires a vital sign from a vital sign measurement apparatus that obtains the vital sign from a subject, said vital sign acquisition apparatus includes: a designated condition creation unit operable to create a designated condition for measuring the vital sign using said vital sign measurement apparatus; an output unit operable to output the designated condition; an input unit operable to obtain, from said vital sign measurement apparatus, the vital sign measured by the subject using said vital sign measurement apparatus, and a measurement condition at a time the vital sign is measured; and a condition comparison unit operable to compare the designated condition and the measurement condition, wherein said output unit is operable to output a result of the comparison made by said condition comparison unit.

According to this structure, it is possible to notify, as a designated condition, the measurement condition that complies with the type of the vital sign and the purpose of the processing result thereof, to the subject. By notifying the designated condition to the subject in this manner, the subject is able to know the designated condition in advance and gets a better understanding regarding the measurement, in addition, the preparations related to the measurement are made easier, and it becomes easier to have the measurement performed according to the designated condition created by the vital sign acquisition apparatus.

Furthermore, by comparing the measurement condition during the actual measurement using the vital information measurement unit, with the designated condition, and outputting the result of the comparison, the subject is able to recognize whether or not the vital sign is measured according to the designated condition. Accordingly, as the subject recognizes the importance of the designated condition and the willingness of the subject to accurately measure the vital sign in accordance with the designated condition, an improvement in the reliability of the vital sign measured by the subject is brought about.

The vital sign acquisition apparatus may further include a compliance level creation unit operable to create a compliance level for the designated condition, based on the result of the comparison made by said condition comparison unit, wherein said output unit may be operable to output the compliance level created by said compliance level creation unit.

According to the present structure, it becomes possible, not just to indicate the result of the comparison between the designated condition and the measurement condition, but to specifically present whether or not the vital sign is measured according to the designated condition, and it becomes easier to recognize and verify whether or not an accurate measurement, which yields information that is useful to the subject, is achieved. Furthermore, by quantifying the compliance level, the compliance level becomes a guide to the assessment of reliability.

The vital sign acquisition apparatus may further include: an information accumulation unit operable to store the vital sign and the measurement condition in association with each other; and an accumulation judgment unit operable to judge, based on the compliance level, whether or not to accumulate the vital sign and the measurement condition, in said information accumulation unit.

According to the present structure, it is possible to accumulate only a vital sign having a high compliance level with respect to the designated condition, and highly useful information can be obtained. Accordingly, accumulation of useful and highly reliable vital signs is made possible, which is conducive to the construction of a database that can better contribute to health management.

In order to solve the existing problem, the vital sign processing method includes the steps of: notifying a subject of a designated condition for measurement of a vital sign by the subject; measuring the vital sign of the subject; recognizing, without input from the subject, a measurement condition at a time of said measuring of the vital sign; comparing the designated condition with the measurement condition; and presenting a result of said comparison.

In order to solve the existing problem, the present invention relates to a vital sign processing program that causes a computer to execute the steps included in the abovementioned vital sign processing method.

### FURTHER INFORMATION ABOUT TECHNICAL BACKGROUND TO THIS APPLICATION

The disclosure of Japanese Patent Application No. 2004-093582 filed on March 26, 2004 including specification, drawings and claims is incorporated herein by reference in its entirety.

### BRIEF DESCRIPTION OF THE DRAWINGS

These and other objects, advantages and features of the invention will become apparent from the following description thereof taken in conjunction with the accompanying drawings that illustrate a specific embodiment of the invention. In the Drawings:
FIG. 1 is a block diagram showing a conventional data acquisition system and data acquisition method.
FIG. 2 is a block diagram showing the structure of a wrist blood pressure monitor which is the vital sign processing apparatus in the embodiment of the present invention.
FIG. 3 is a schematic diagram showing an example of the vital sign measurement device used in the present embodiment.
FIG. 4 is a diagram showing a screen sample of the display screen included in the subject notification unit.
FIG. 5 is a diagram showing the processing operation and the flow of data, after the vital sign processing apparatus 101 receives the designated condition.
FIG. 6 is a diagram showing an example of compliance level creation.
FIG. 7 is a block diagram showing the vital sign processing system in the embodiment of the present invention.
FIG. 8 is a schematic diagram showing the appearance of the blood pressure monitor which is the portable measurement unit, being worn by a person.
FIG. 9 is a schematic diagram showing the appearance of the display of the subject notification unit.
FIG. 10 is a diagram showing the processing operation and the flow of data, up to the creation of designated condition by the vital sign acquisition apparatus.
FIG. 11 is a diagram showing the processing operation and the flow of data, for the vital sign measurement apparatus.
FIG. 12 is a diagram showing the processing operation and the flow of data, for the vital sign measurement apparatus.
FIG. 13 is a diagram showing information display operation and the flow of data, for the vital sign measurement apparatus.
FIG. 14 is a schematic diagram showing the appearance of another display of the subject notification unit.
FIG. 15 is a graph indicating the circadian variation of the blood sugar level
FIG. 16 is a diagram showing, by chemical formula, the changing of dG and 8-OHdG.

### DESCRIPTION OF THE PREFERRED EMBODIMENT(S)

Hereinafter, the embodiments of the present invention shall be explained with reference to the diagrams.

### (First Embodiment)

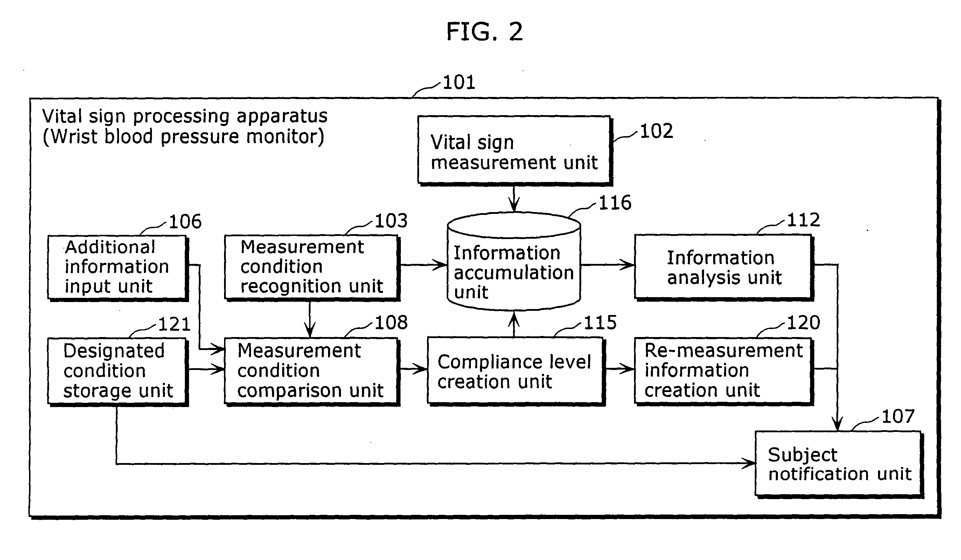

FIG. 2 is a block diagram showing the structure of a wrist blood pressure monitor which is a vital sign processing apparatus 101 in an embodiment of the present invention.

This blood pressure monitor is an apparatus that measures blood pressure by being wrapped around a wrist, and applying pressure to the wrist. It generally includes a vital sign measurement unit 102, a measurement condition recognition unit 103, an additional information input unit 106, a subject notification unit 107 functioning as a designated condition notification means, as well as a presentation means, a designated condition storage unit 121, a measurement condition comparison unit 108, a compliance level creation unit 115, an information accumulation unit 116, a re-measurement information creation unit 120, and an information analysis unit 112.

The vital sign measurement unit 102 is structured from a pulse sensor for detecting pulse information which is pulsation information of the heart, and a converter and micro processing unit (MPU) that perform the analysis of an output signal of the pulse sensor.

The measurement condition recognition unit 103 is structured from a sensor for detecting a measuring posture, and a timing unit.

A sensor that can detect the height of the pulse measuring position is sufficient as the sensor for detecting the measuring posture. Although a sensor using an inclination sensor and heart sound sensor is given as an example, the present embodiment does not particularly restrict the type used. Aside from the date and time at which a measurement is taken, the timing unit can also generate, as a measurement condition, the amount time spent for the measurement.

The additional information input unit 106 is a processing unit through which the subject inputs information that cannot be measured by the measurement condition recognition unit 103. For example, information such as "the time a meal is taken", "the contents of a meal", "busy at work", "feeling fatigue", and "whether one has smoked or not" can be inputted using the additional information input unit 106.

The designated condition storage unit 121 is a processing unit for storing a condition to be measured by the vital sign processing apparatus 101 as a designated condition, and is a storage medium such as a ROM.

Designated condition refers to a measurement condition that needs to be measured by the vital sign processing apparatus 101, and is, for example, information regarding measurement such as the item to be measured, the frequency and interval of measurement, the reagent used and the setting condition of the measuring device.

In the present embodiment, the designated condition is the measuring posture at the time of blood pressure measurement, and adopts the structure of a program instructing measurement to be performed with the measurement apparatus positioned at the same height as the heart.

The designated condition storage unit 121 stores the designated condition and the program for controlling the vital sign processing apparatus 101 so as to measure according to the designated condition. In addition, the designated condition is displayed to the subject through the subject notification unit 107 which serves as a presentation means.

The subject notification unit 107 is a processing unit serving as a presentation means for notifying the designated condition to the subject. It is possible to have a text and graphic display, and a notification by sound and light, as a specific notification means.

The measurement comparison unit 108 is a processing unit for comparing measurement conditions and additional information during the actual measurement of the vital sign, with the specified designated condition, and judging whether or not the subject has taken the measurements in accordance with the designated condition.

In the present embodiment, the measurement condition comparison unit 108, in addition, compares the additional information by obtaining a designated condition input from the designated condition storage unit 121, and obtaining a measurement condition input from the measurement condition recognition unit 103.

The compliance level creation unit 115 is a processing unit for creating, with respect to the comparison result from the measurement condition comparison unit 108 and other items, a compliance level for whether or not the subject has taken the measurement according to the designated condition.

The compliance level indicates whether or not the subject has taken the measurement according to the designated condition, and is created by judging whether or not the subject has complied with the designated condition by comparing measurement conditions and additional information during the actual measurement of vital signs, with the designated condition. At this time, it is also possible to create the compliance level coupled with the vital sign of the actual measurement. For example, the compliance level is low when the vital sign of the actual measurement is far off from a normal value.

The re-measurement information creation unit 120 is a processing unit which sets an appropriate lower limit for the compliance level and creates re-measurement information when the lower limit of the compliance level is not reached, as the reliability of such measurement becomes low in the case where the compliance level is too low. In the case where such re-measurement information is created, it is notified to the subject through the subject notification unit 107.

The information accumulation unit 116 is a nonvolatile memory such as a hard disk apparatus, for associating and storing the measurement conditions and the vital sign. Furthermore, the information accumulation unit 116 can also adopt a form in which, aside from the abovementioned information, personal information and additional information, the compliance level, and analysis information, are respectively associated and stored.

The information accumulation unit 116 includes an accumulation judgment means not shown in the diagram. This accumulation judgment means is a processing unit which judges a compliance level, and accumulates, in the information accumulation unit 116, only vital signs and information related thereto, that meet a fixed compliance level.

The information analysis unit 112 is a processing unit which analyzes vital signs, based on the measurement condition, additional condition, and so on, and creates corresponding medical information.

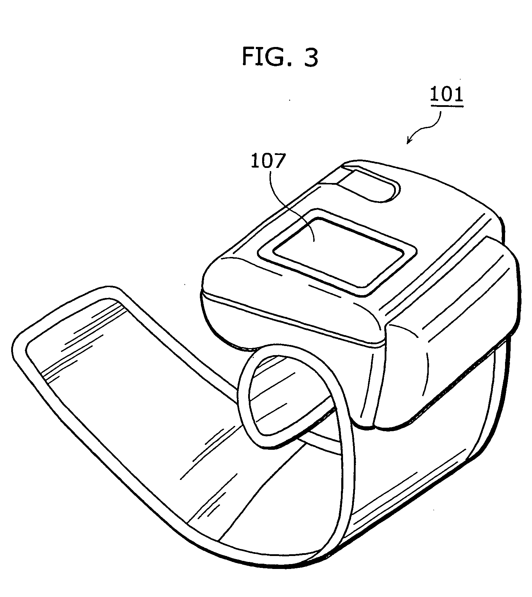

FIG. 3 is a schematic diagram showing an example of a vital sign measurement device used in the present embodiment. It is a wrist blood pressure monitor having a function for measuring blood pressure by wrapping a measuring device around the wrist, and displaying such measurement value on a subject notification unit 107.

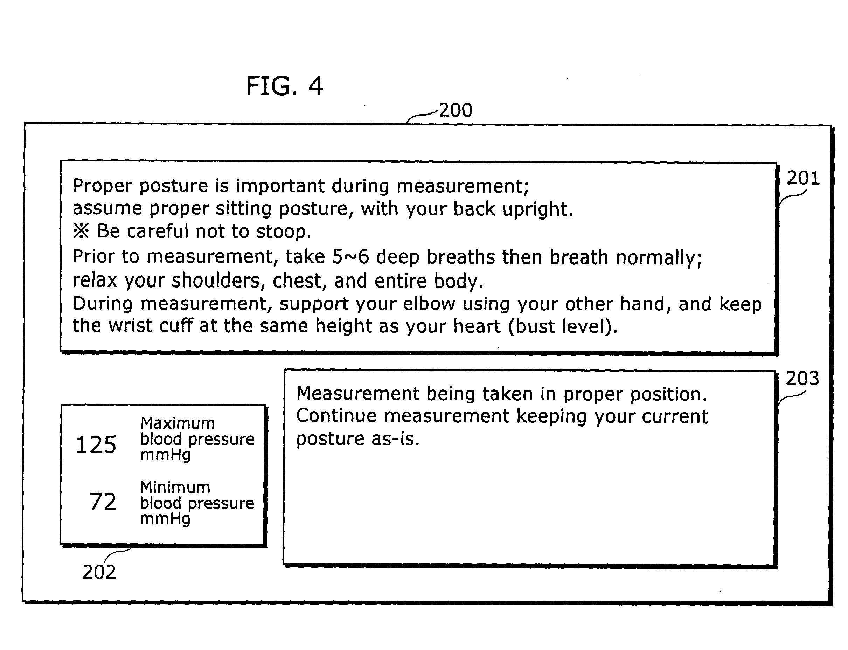

FIG. 4 shows a display screen 200 included in a subject notification unit 107. The display screen 200 includes a designated condition display unit 201, a measurement value display unit 202, and an information display unit 203.

The designated condition display unit 201 is a display unit for displaying designated conditions. In the present embodiment, it indicates a posture and a height of the wrist blood pressure monitor to be assumed during measurement, and a method for attaining a relaxed state.

The measurement value display unit 202 is a display unit for displaying an actual measurement value measured by the vital sign measurement unit 102.

The information display unit 203 is a display unit for displaying a compliance level for a designated condition, and so on.

Next, the procedure for the processing of vital signs in the present embodiment shall be explained, using the procedure for measuring blood pressure for the blood pressure management of a subject as an example.

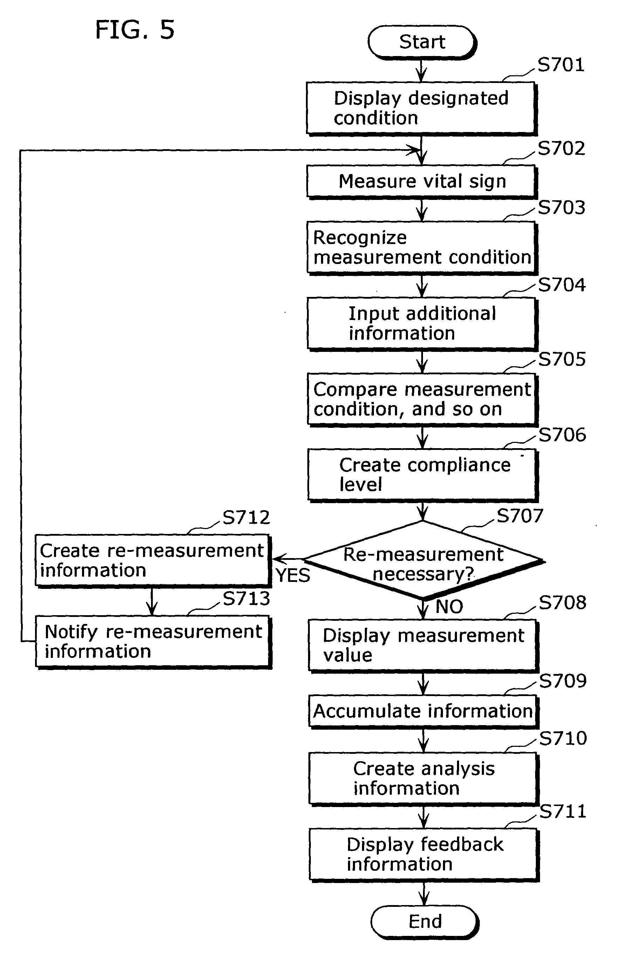

FIG. 5 is a diagram showing the processing operation and flow of data after the vital sign processing apparatus 101 receives designated conditions. The designated condition includes the proper posture during blood pressure measurement, specifically, an instruction to take the measurement by positioning the blood pressure monitor, which is wrapped around the wrist, at the same height as the heart.

In the vital sign processing apparatus 101, the designated condition stored in the designated condition storage unit 121 is displayed on the designated condition display unit 201 (S701).

With this display, the subject can be prompted to take the measurement according to the designated condition. Furthermore, as the subject is notified of the designated condition in advance, the subject has a better understanding of the measurement condition and so on. In addition, the preparations related to the measurement are made easier, and it becomes easier to have the measurement performed under the designated condition.

Next, the subject measures blood pressure as the vital signs using the vital sign measurement unit 102 (S702). At the same time, recognition of the measurement conditions during the blood pressure measurement is also automatically performed (S703). Through the sensor, the height of the pulse measurement position is detected. In addition, measurement conditions such as the date and time, atmospheric temperature, and humidity measured are created in accordance with the measurement.

After these measurements, additional information is inputted (S704). Examples are "whether or not the subject is rested", "whether or not the subject lacks sleep", and so on. With the input of such additional information, it becomes possible to get a more detailed understanding as to the environment and situation under which the measured blood pressure value was obtained.

The measurement condition and the additional information are compared with the designated condition (S705). For example, a check is made as to whether the measurement was performed with the pulse measurement position at heart-level height as specified by the designated condition. Specifically, the inclination of the wrist blood pressure monitor can be obtained from an inclination sensor provided in the vital sign processing unit 101. From such inclination, it is possible to infer that the arm on which the blood pressure monitor is attached is bent at a prescribed angle. Subsequently, the approximate positions of the heart and the wrist can be inferred from the angle at which the arm is bent.

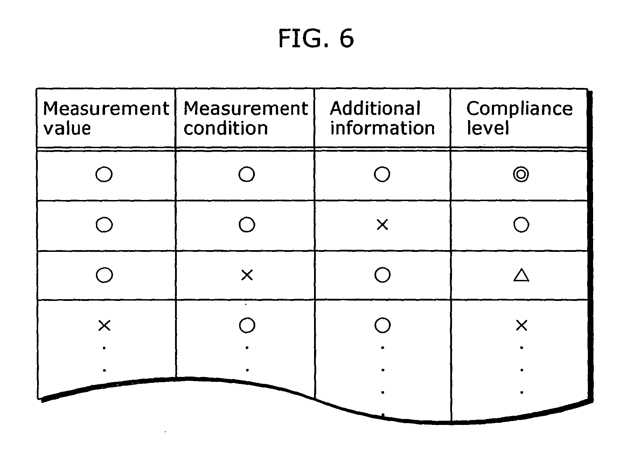

Next, the compliance level creation unit 115 receives the result of this comparison, and creates a compliance level (S706). The compliance level, as shown in FIG. 6 for example, evaluates whether or not the measurement value is a commonsense value, and gives a graduated rating of how much the measurement condition and additional information differ from the designated condition. These are calculated together.

When the compliance level is poor, as in the case where measurement is not performed at the correct measurement position or when the measurement value is far from normal, for example (YES in S707), re-measurement information is created by the re-measurement information creation unit 120 (S712), and notified through the subject notification unit 107 (S713). In other words, in the case where blood pressure is not measured in the correct posture, information indicating a need for re-measurement is created. A message such as, "Measurement posture is incorrect. Re-measurement is necessary. Set position of blood pressure monitor at heart-level height and perform blood pressure measurement again." are displayed on the subject notification unit 107.

Subsequently, in this case, re-measurement of the vital signs is performed automatically (S702).

On the other hand, when the compliance level is good and re-measurement is not necessary (NO in S707), the measurement value is displayed in real-time, on the measurement value display unit 202 (S708).

Next, as only information having a good compliance level is obtained in the case of the present embodiment, such information is accumulated in the information accumulation unit 116 (S709). In the information accumulation unit 116, the blood pressure which is the vital sign, the measurement conditions, and the additional information, together with previously inputted information on the subject such as gender, age, and occupation are associated with each other and stored.

Next, the measurement analysis unit 112 extracts, from the information accumulation unit 116, the vital signs and the associated measurement condition, and creates analysis information (S710). Furthermore, a feedback information creation unit (not shown in the diagram) creates feedback information based on the compliance level, the analysis result from the information analysis unit 112, and so on, and such information is also accumulated in the accumulation unit. In the end, this information can be summoned by button operation and displayed on the display unit (S711).

According to the vital sign processing apparatus described above, the subject is able to measure the vital signs according to correct measurement conditions, and only correct vital signs are accumulated together with the measurement condition, and so on. Accordingly, by analyzing these, it becomes possible to ascertain the correct physical condition.

Furthermore, for example, if billing information which is the feedback information decreases as the measurement becomes more accurate, the motivation for taking measurements more accurately can be increased.

Moreover, by leaving such blood pressure monitor with a prescribed institution, and giving out gift certificates to the subject based on the billing information which is the feedback information from the accumulated data acquisition of the institution, the institution which is the acquiring-side can acquire accurate information, and the subject can receive gift certificates, and the like, thereby proving to be effective.

### (Second Embodiment)

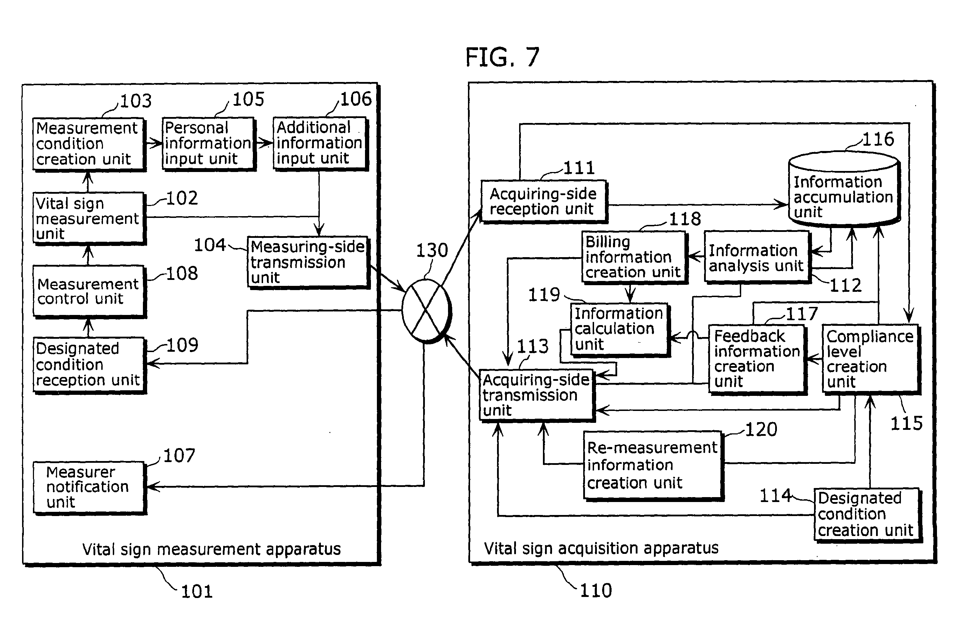

FIG. 7 is a block diagram of the vital sign processing system in an embodiment of the present invention. The vital sign processing system includes a plurality of vital sign measurement apparatuses 101, and a vital sign acquisition apparatus 110 connected via a communication network 130 which serves as an intermediary. It is a system capable of extensively acquiring and processing vital signs. Furthermore, although a single vital sign measurement apparatus 101 is shown in FIG. 7, other vital sign measurement apparatuses 101 not shown in the diagram, have the same structure and are connected to the communication network 130.

The vital sign measurement apparatus 101 is located near the subject and is an apparatus for transmitting, to the vital sign acquisition apparatus 110, vital signs and measurement conditions at the time of the measurement. It generally includes a vital sign measurement unit 102, a measurement condition creation unit 103, a measuring-side transmission unit 104, a designated condition reception unit 109, a subject notification unit 107, a personal information input unit 105, an additional information input unit 106, and a measurement control unit 108.

The vital sign measurement unit 102 includes a measurement device that is not illustrated in the diagram, and measures the vital sign of a subject. The type of vital sign to be measured is not restricted, and for example, aside from the weight, body temperature, body fat, blood pressure, and so on, can also be a vital sign obtained from excrement such as urine. Accordingly, a weighing scale, a thermometer, a body fat monitor, a blood pressure monitor, a urine analyzer, a fecal viscometer, and so on, are given as measurement devices for directly measuring such vital signs.

The measurement condition creation unit 103 is a processing unit serving as a measurement condition recognition means for automatically creating a measurement condition associated with the measured vital signs. The measurement condition creation unit 103 includes a timing unit that is not shown in the diagram. Aside from the date and time on which a measurement was taken, the timing unit can also generate, as information, the amount of time spent for the measurement.

The personal information input unit 105 is a processing unit including a man-machine interface, which creates personal information through the input of information that identifies a person such as gender, age, and occupation.

The additional information input unit 106 is a processing unit through which the subject inputs vital signs that cannot be measured by the vital sign measurement unit 102, and information that cannot be created by the measurement condition creation unit 103. For example, information such as "the time a meal is taken", "the contents of a meal", "busy at work", "feeling fatigue", and "whether one has smoked or not" can be inputted to the additional information input unit 106.

The additional information input unit 106 includes a display unit, and a corresponding selection unit, which are not shown in the diagram, and can create additional information merely through the selection of information displayed in the display unit, in the corresponding selection unit. In addition, such displayed information is changed in accordance with a designated condition to be described later, using an entry changing means not shown in the diagram.

The measuring-side transmission unit 104 is a processing unit serving as a measurement apparatus-side transmission means for associating the vital sign and measurement condition, personal information, and additional information with each other and transmitting such associated information to the vital sign acquisition apparatus 110.

The designated condition reception unit 109 is a processing unit serving as a measurement apparatus-side input means for receiving a designated condition transmitted by the vital sign acquisition apparatus 110.

The measurement control unit 108 is a processing unit for controlling the vital sign measurement unit 102 based on the received designated condition, and is implemented through a program for controlling the vital sign measurement unit 102.

The subject notification unit 107 is a processing unit for notifying the designated condition transmitted by the vital sign acquisition apparatus 110 to the subject. As a specific means for notification, it is possible to have a text and graphic display, and a notification by sound and light. In addition, it is also possible to have a means that notifies the subject by obtaining the designated condition through a means such as electronic mail or facsimile. Furthermore, aside from the case in which the subject notification unit 107 is provided in the vital sign measurement apparatus 101, it also possible to use a mobile phone which the subject carries around as the subject notification unit 107.

On the other hand, the vital sign acquisition apparatus 110 receives the vital signs, and the like, transmitted from the plurality of vital sign measurement apparatuses 101, and processes such transmitted information. It generally includes an acquiring-side reception unit 111 serving as an acquisition apparatus-side input means, an information analysis unit 112, an acquiring-side transmission unit 113 serving as an acquisition apparatus-side output means, a designated condition creation unit 114, a compliance level creation unit 115, an information accumulation unit 116, a re-measurement information creation unit 120, a feedback information creation unit 117, a billing information creation unit 118, and an information calculation unit 119.

The acquiring-side reception unit 111 is a processing unit for receiving the vital signs, and the like, and is connected to the communication network 130.

The designated condition creation unit 114 is a processing unit for creating measurement conditions that need to be measured by the vital sign measurement apparatus 101, and so on. It creates, for example, designated information such as the item to be measured, the frequency and interval of measurement, the reagent used and the setting condition of the measuring device.

The compliance level creation unit 115 is a processing unit for creating the compliance level with respect to whether or not the subject carried out the measurement according to the designated condition, by comparing the measurement condition actually measured with the specified designated condition, and also considering the additional information. The compliance level indicates whether or not the subject has taken the measurement according to the designated condition, and is created by judging whether or not the subject has complied with the designated condition by comparing the measurement conditions of the vital signs that are actually measured, with the specified designated condition. At this time, it is also possible to create the compliance level coupled with the vital signs of the actual measurement.

The re-measurement information creation unit 120 is a processing unit which sets an appropriate lower limit for the compliance level and creates re-measurement information when the lower limit of the compliance level is not reached, as the reliability of such measurement becomes low in the case where the compliance level is too low. When such re-measurement information is created, it is notified to the subject notification unit 107 using the acquiring-side transmission unit 113 described later.

The information accumulation unit 116 is a nonvolatile memory such as a hard disk apparatus, for associating and storing the measurement condition and vital signs. Furthermore, aside from the previously mentioned information, the information accumulation unit 116 stores the personal information and additional information, the compliance level, analysis information, billing information and feedback information to be described later, which are associated with the measurement condition and vital signs.

The information accumulation unit 116 includes an accumulation judgment means not shown in the diagram. This accumulation judgment means is a processing unit which judges the compliance level, and accumulates, in the information accumulation unit 116, only vital signs and information related thereto, that meet a fixed compliance level.

The information analysis unit 112 is a processing unit which analyzes vital signs based on the measurement condition, additional condition, and so on, and creates corresponding medical information, and so on.

The feedback information creation unit 117 is a processing unit for creating feedback information in accordance to the compliance level created by the compliance level creation unit 115. As the compliance level increases, feedback information that is advantageous to the subject is created. Furthermore, the feedback information can also be "point" information that is on a one-to-one correspondence to a sum of money necessary to purchase a product or receive a service being provided.

The feedback information creation unit 117 includes an information selection means not shown in the diagram. The information selection means enables the subject to appropriately set the details of the product or service applicable to the feedback information, and can be set through the personal information input unit 105. Aside from measurement related products such as reagents, and services such as the provision of analysis information, it is also possible to have as products and the like that are applicable as feedback information, items not relating to vital sign measurement such as "miles" for mileage programs, marketable securities, and weather forecasting services. In addition, it is also possible to enable such "points" to be converted to other point systems such as electronic money, and so on.

The billing information creation unit 118 is a processing unit for creating, as the billing information, expenses involved in the accumulation and analysis of the vital signs, and the like.

The information calculation unit 119 is a processing unit for calculating the billing information and the point information.

The acquiring-side transmission unit 113 is a processing unit for transmitting, via the communication network 130, the designated condition to the designated condition receiving unit 109 and subject notification unit 107. Furthermore, aside from the designated condition, it also has a combined a function for transmitting the compliance level, feedback information, analysis information, billing information, calculation results from the information calculation unit 119, and so on.

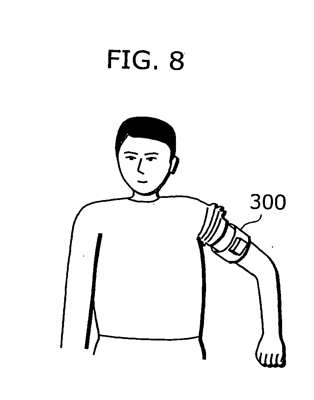

FIG. 8 is a schematic diagram showing an example of a measurement device included in the vital sign measurement unit 102. A portable measurement unit 300 is a blood pressure monitor having the portability of being fastened, in a wrap-around manner, onto a human arm. This blood pressure monitor transmits measurement values to the vital sign measurement apparatus 101 by a wireless link.

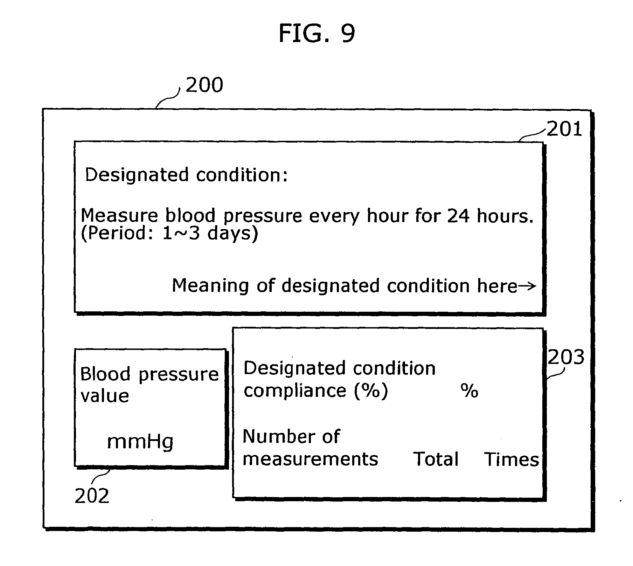

FIG. 9 shows a display screen 200 included in the subject notification unit 107. The display screen 200 includes a designated condition display unit 201, a measurement value display unit 202, and an information display unit 203 on which the compliance level, and so on, are displayed.

The designated condition display unit 201 is a display unit for displaying a designated condition transmitted from the designated condition creation unit 114.

The measurement value display unit 202 is a display unit for displaying an actual measurement value measured by the vital sign measurement unit 102.

The information display unit 203 is a display unit for displaying a compliance level, and the number of measurements remaining designated in the designated condition, transmitted from the vital sign acquisition apparatus 110.

Next, the procedure for the processing of vital signs in the vital sign processing system shall be explained, using as an example, the procedure for measuring and processing a circadian variation of blood pressure for the blood pressure management of a subject.

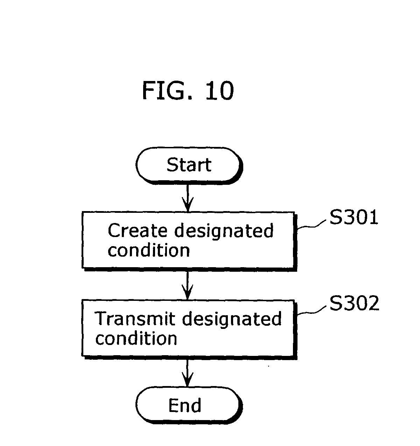

FIG. 10 is a diagram showing the designated condition creating operation and flow of data in the vital sign acquisition apparatus 110.

The designated condition creation unit 114 of the vital sign acquisition apparatus 110 creates a designated condition needed to be measured by the vital sign measurement apparatus 101 (S301), and transmits such designated condition to the designated condition reception unit 109 and the subject notification unit 107 (S302). Furthermore, the designation condition can also be created automatically from past data accumulated in the information accumulation unit 116, and in addition, can also be arbitrarily created through an input means that is not shown in the diagram.

The designated condition includes information telling the subject to check blood pressure circadian variation in advance, to wear the blood pressure monitor which is the portable measurement unit 300 continually for 3 days, and information for operating the measurement control unit 108 so as to measure blood pressure at fixed intervals.

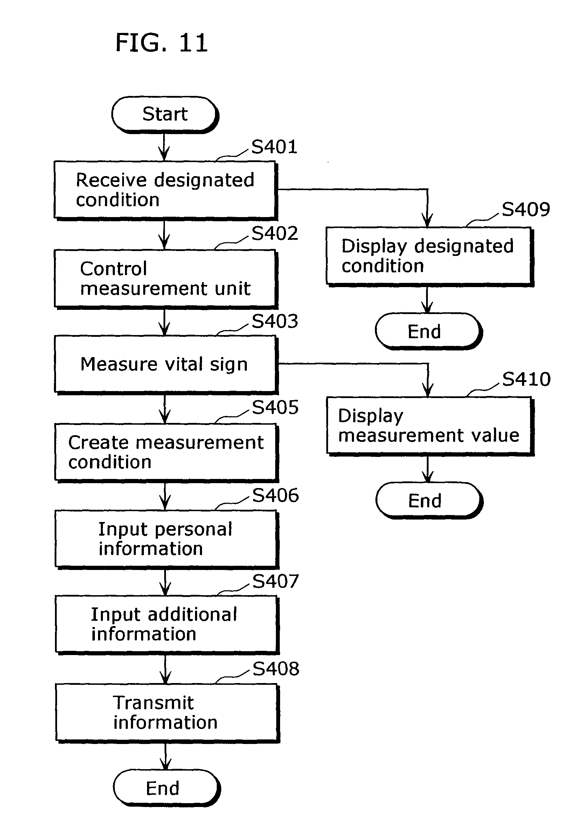

FIG. 11 is a diagram showing the processing operation and flow of data after the vital sign measurement apparatus 101 receives a designated condition.

At the vital sign measurement apparatus 101-side, the designated condition reception unit 109 and the subject notification unit 107 receive the designated condition (S401).

In accordance with the reception of the designated condition, the measurement control unit 108 controls the vital sign measurement unit 102, and setting of measurement condition, and so on, is performed (S402).

Also in accordance with the reception of the designated condition, the designated condition is displayed on the designated condition display unit 201 (S409). With this display, the subject can be prompted to perform the measurement according to the designated condition, and the subject can wear the blood pressure monitor 300 in advance. Furthermore, as the subject is notified of the designated condition in advance, the subject has a better understanding of the measurement condition and so on, in addition, the preparations related to the measurement are made easier, and it becomes easier to have the measurement performed under the designated condition.

Next, the subject measures the vital signs using the vital sign measuring unit 102 (S403). As the blood pressure monitor 300 is controlled by the measurement control unit 108 so as to measure blood pressure at fixed intervals, apart from when the subject removes the blood pressure monitor 300 or an anomaly such as a malfunction arises, the vital signs are measured according to the designated condition and the measurement value is transmitted to the vital sign measurement apparatus 101.

When the blood pressure is measured, its value is displayed in real-time on the measurement value display unit 202 (S410).

In accordance with this measurement, the measurement condition creation unit 103 creates the measurement conditions such as measured date and time, atmospheric temperature, humidity and so on (S405).

After the conclusion of the measurement sequence, the subject inputs his personal information using the personal information input unit 105 (S406), and in addition, inputs additional information (S407). With the input of such personal information and the input of such additional information, it becomes possible to get a more detailed understanding as to the environment and situation under which the measured blood pressure value was obtained.

Finally, the measuring-side transmission unit 104 transmits all the above information to the vital sign acquisition apparatus 110 (S408).

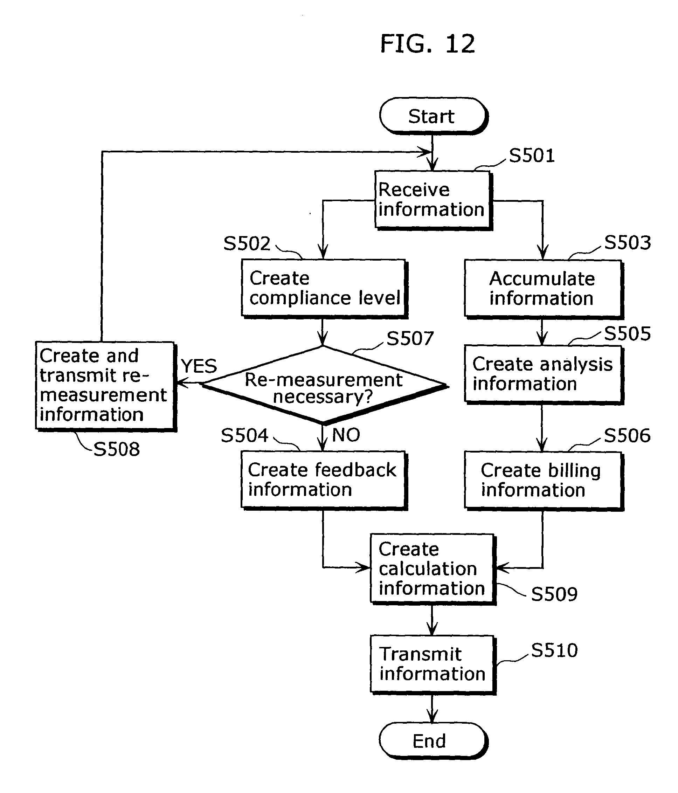

FIG. 12 is a diagram of the processing operation and flow of data after the vital sign acquisition apparatus 110 receives the various information such as the vital sign.

In accordance with the reception of the information such as the vital sign (S501), the compliance level creation unit 115 compares the received measurement conditions with the pre-specified designated condition, and creates a compliance level (S502). In the case of the present embodiment, it is judged that designated condition compliance level is 100% when it can be verified that blood pressure was measured continually at fixed time intervals for one day. Furthermore, the compliance level is judged to be 200% when continual measurement extends to 2 days, and the compliance level is judged as being 300% when measurement can be taken continually for 3 days. In other words, assuming the period of time over which blood pressure is continually measured is "X", and the designated condition is that measurements are taken once every hour, the compliance level can be calculated through the formula, { (X - 24) / 24 + 1} × 100 (%). In addition, in the case where the measurement value of the vital signs deviate greatly from the range that is possible for a living body, and so on, even though the compliance level derived using the formula is a high value, a process which lowers the compliance level is taken.

Subsequently, in the case where the compliance level does not reach a fixed value (YES in S507), re-measurement information is created by the re-measurement information creation unit 120, and this information is transmitted to the subject notification unit 107 (S508). In other words, as data for checking the circadian variation of blood pressure is insufficient in the case where the number of times that blood pressure is measured does not meet predetermined number of times, information stating that there is a need for re-measurement is created. The subject notification unit 107 receiving such information displays a message such as, "Circadian variation of blood pressure cannot be checked due to insufficient data. Re-measurement is required. Check if measurement device is operating properly; wear blood pressure monitor continually for 1 day or more, up to 3 days; and transmit the data".

Next, in the case where there is no need for re-measurement (NO in S507), feedback information is created in accordance with the compliance level (S504).

On the other hand, the information transmitted from the vital sign acquisition apparatus 110 is accumulated by the information accumulation unit 116 (S503). The information accumulation unit 116 accumulates vital signs according to the respective information of the subject such as gender, age, occupation, which is inputted in advance.

The information analysis unit 112 extracts the vital signs and the associated measurement conditions from the information accumulation unit 116, and creates analysis information (S505). Subsequently, the billing information creation unit 118 creates billing information based on the accumulated vital signs and the analysis information from such analysis (S506).

Next, the information calculation unit 119 carries out the calculation of the billing information and the feedback information, and creates calculation information (S509). Finally, the analysis information, the billing information, the feedback information, and the calculation information, are transmitted by the acquiring-side transmission unit 113 to the subject notification unit 107 (S510).

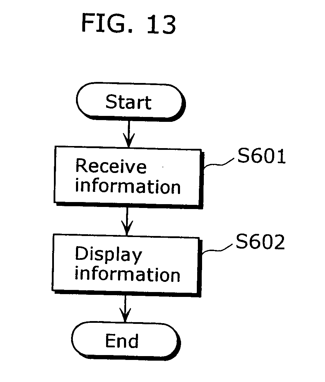

FIG. 13 is a block diagram showing the processing operation of the vital sign measurement apparatus 101 after receiving the various information created by the vital sign acquisition apparatus 110.

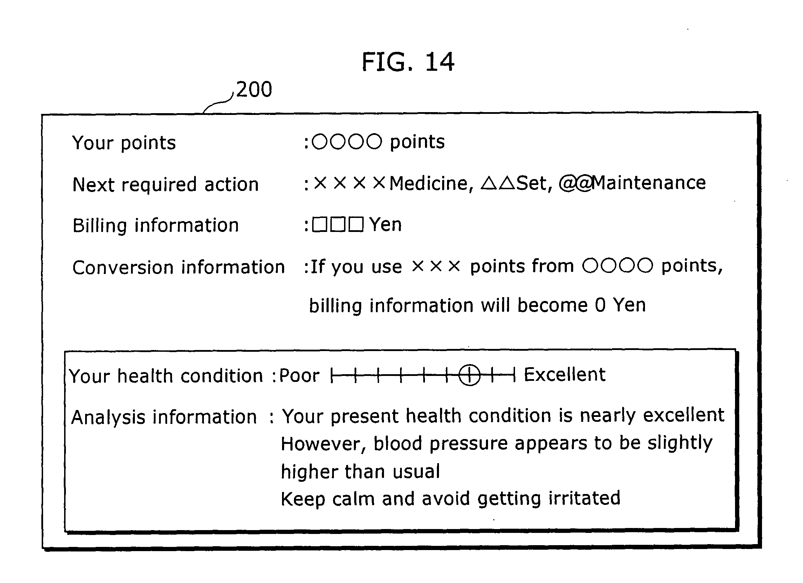

The various information are received by the subject notification unit 107 (S601), and information such as the compliance level and the number of measurements remaining that are required from here on, is displayed on the display screen 200 shown in FIG. 14 (S602). As shown in FIG. 14, by showing the feedback information (point information) and the analysis information obtained from the compliance level, the result obtained by following the designated condition can be readily ascertained, and this encourages compliance to the designated conditions on the next measurement.

Furthermore, as shown in FIG. 14, the billing information, the analysis information, and so on, are also displayed together in the display screen 200 of the subject notification unit 107. The content of analysis information is not restricted to matters concerning the measurement value, and is set as may be deemed fit. It can also be information regarding health information in accordance with the season such as hay-fever information and communicable disease information, illnesses, and medicines.

According to the aforementioned vital sign processing system, the subject is not restricted to merely providing his own vital signs and having it managed, but it is also possible for him to obtain more useful and highly reliable information. In addition, when measurement of vital signs is performed properly according to the designated condition, it is possible to obtain point information. By offsetting, with the point information, the expenses required in obtaining the aforementioned useful information, purchasing reagents and maintenance products needed for the measurement device, and so on, it becomes possible to obtain the aforementioned information and products at cheaper prices or for free. Furthermore, with this, an effect of having the subject perform the measurement of the vital signs more accurately can be produced.

On the other hand, by previously providing the conditions to be measured, as designated condition, to the subject, the information acquiring-side is able to adequately inform the subject of the significance and the details of the vital sign measurement. In addition, as adequate preparation for measurement can be encouraged, the subject can be expected to measure vital signs accurately. Furthermore, as the actual measurement condition is compared with the specified designated condition, with the result being shown to the subject, and in addition, as higher point information is added the more the designated condition is complied with, the subject can be expected to measure vital signs more accurately. In addition, aside from being useful in analyzing the health condition of the subject, vital signs based on accurate conditions improve the reliability of the acquired data, and enables the construction of a database that can better contribute to health management. Such database can be managed on a per-subject basis, and can also be used as a basis for widespread provision of information.

Moreover, although explanation in the present embodiment is carried out using the communication network as an example of an intermediary, it is also possible to have a portable storage medium as the intermediary.

Furthermore, although the case where the personal information input unit 105 and the additional information unit 106 are included in the vital sign measurement apparatus 101 is exemplified, it is also possible to have the personal information input unit 105 and the additional information unit 106 included in the vital sign acquisition apparatus 110 only, or included in both. In particular, it is also possible to have an expert's opinion written in through the additional information input unit 106.

Furthermore, it is also possible to prevent viewership by someone without viewing authority by having the information accumulation unit 116 carry a verification function, and providing a security such as a password.

### (Third Embodiment)

Next, explanation shall be made regarding an embodiment using a vital sign measurement unit 102 which includes a blood sugar monitor. Furthermore, explanation regarding units having the same function and the same operation as in the previous embodiments shall be omitted.

Blood sugar level varies constantly within the course of a day depending on meals, exercise, and so on. However, for a normal healthy subject, the blood sugar level is regulated within a certain fixed range (60~109mg/dl fasting, below 140mg/dl two hours after a meal). Although normally the standard range for fasting blood sugar level is used as an assessment of blood sugar, there are cases where post-meal blood sugar has a high blood sugar level even when the fasting blood sugar is normal, and it is necessary to keep track of circadian variation in determining a course of treatment for diabetes.

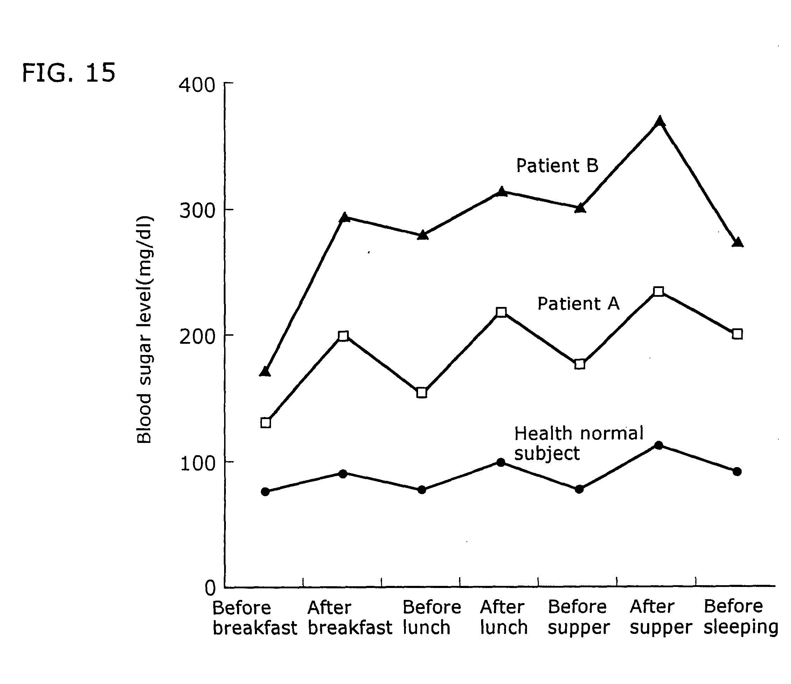

FIG. 15 is a graph showing respective circadian variation patterns of a normal healthy subject, a patient A observed as having high post-meal blood sugar, and a patient B with a continuing condition of having high blood sugar overall.

Although there are many types of blood sugar measuring methods, these can be largely divided into two types, namely an enzymatic colorimetric method and an enzymatic electrode method. The enzymatic colorimetric method is a method where glucose is made to react with an oxidizing enzyme and a visible light ray is applied onto the final product, and the decrease in the light volume in the reflected light is measured by a sensor. The enzymatic electrode method is a method where glucose is made to react with an oxidizing enzyme or a dehydrogenated enzyme, and the "e-" of the final product is measured, as a value of a current, using an anode. The present embodiment does not particularly restrict the blood sugar level measurement method.

A designated condition creation unit 114 transmits, to a subject notification unit 107 via a communication network 130, a designated condition stating, "In order to check circadian variation of blood sugar level, measure blood sugar level before meals, 2 hours after meals, and before sleeping - seven times in total" (S301, S302).

With each measurement of blood sugar, the subject inputs the blood sugar level measurement conditions before meals, after meals, and before sleeping, using the input means of the additional information input unit 106 (S403, S405, S407). The inputting method is not particularly restricted, and can be a method where a button, provided for each measurement condition, is pressed, or it is also possible to input by text or sound.

The subject notification unit 107 also includes a function for notifying the subject at a predetermined time using a buzzer, and the like, in accordance with the designated condition. With this notification, the subject can be given a rough indication for the timing of the measurements, and acquisition of more accurate vital signs is made possible.

Next, a measuring-side transmission unit 104 stores the data of the blood sugar level to be transmitted to the vital sign acquisition apparatus 110, until a predetermined number of times is reached. When the predetermined number of times is reached, the measuring-side transmission unit 104 transmits to the vital sign acquisition apparatus 110 the following: the measurement condition inputted by the subject; the blood sugar level measured by the vital sign measurement unit 102; the blood sugar value collection times obtained using a timing function of a measurement condition creation unit 103; and the personal information. Moreover, it is also possible for the measuring-side transmission unit 104 to transmit such information at every measurement.

A compliance level creation unit 115 of the vital sign acquisition apparatus 110 which has received the information, checks the measurement information and creates the compliance level as to whether or not the designated condition has been complied with (S502). This compliance level is judged as to whether or not blood sugar level is measured for the predetermined number of times, and whether or not it is measured before meals and 2 hours after meals. When both conditions are satisfied, the compliance level is judged as being "○" (good). When it is judged as being "×" (no good), a judgment requiring a re-measurement is given as there is insufficient data for checking the circadian variation of the blood sugar level (S507). The subject notification unit 107 displays a message such as, "Circadian variation of blood sugar level cannot be checked due to insufficient data. Re-measurement is required. Check if the measurement device is operating properly, and transmit data again".

The subject notification unit 107 receiving this notification is a mobile phone which is a unit separated from the vital sign measurement apparatus 101, and the notification to the subject is carried out through electronic mail.

At the same time, an information analysis unit 112 analyzes the blood sugar level transmitted from the vital sign measurement apparatus 101 (S505). It judges a pre-meal blood sugar level of under 110mg/dl as normal type, 110mg/dl~126mg/dl as borderline type, and above 126mg/dl as diabetic type, and transmits this as analysis information.

The subject notification unit 107 receives the transmitted analysis information and presents this to the subject (S602). By verifying the transmitted measurement conditions and compliance level, the subject verifies that the specified designated condition has been complied with, and understands that, as a result of the accurate taking of measurements, glucose metabolism irregularity can be judged from the blood sugar level. At this time, information concerning health and illnesses such as diabetes can be coupled with the analysis information and transmitted. In addition, the information analysis unit 112 extracts the blood sugar levels and the data collection times thereof, from the information accumulation unit 116, and transmits these to the subject notification unit 107. The subject notification unit 107 can show these in the form of a graph. From the graph shown, the subject can use the information as a guide for blood sugar control.

By adopting a structure in which the additional information input unit 106 is used by the subject to transmit, as additional data, the contents of a meal and health related data measured through an apparatus other than the vital sign measurement unit 102, together with the blood sugar level, a database made from the vital signs and so on, accumulated in the information accumulation unit 116, is not limited to being merely a record of blood sugar circadian variation, and becomes an enhanced database covering information regarding the subject's health.

Furthermore, it is possible to prevent usage by a person without viewing authority by carrying out the following: distributing a serial number for each subject; transmitting an identification number inputted through the personal information input unit 105 with each transmission of vital signs; accumulating the vital signs together with the identification of such apparatus in the information accumulation unit 116; and providing the information accumulation unit 116 with a verification function and setting a security such as a password.

According to the present embodiment, by explaining the meaning of the measurement using the designated condition, managing the compliance level thereof, and transmitting the compliance level and analysis information together, the subject acquires a better understanding of the measurement, an accurate measurement value complying with a designated condition can be acquired, and with this as a basis, it becomes possible to construct a database necessary for the analysis of the measurement value.

### (Fourth Embodiment)

Next, explanation shall be made regarding an embodiment using the vital sign measurement unit 102 functioning as a "health" toilet, and including a urine measurement unit (not shown in diagrams). Furthermore, explanation regarding units having the same function and the same operation as in the previous embodiments shall be omitted.

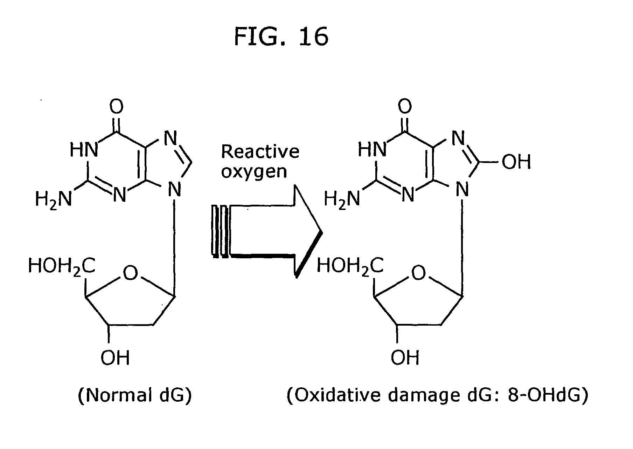

When oxidative stress is aggravated by reactive oxygen that cannot be eliminated through the antioxidation system within the human body, elements important to the body such as fat, protein, enzymes, and DNA, are oxidized. Oxidation damage to such elements is thought to bring about a reduction in cellular functions, mutation of genes, and so on, and as a result, is linked to aging and illness. Genes are constructed from four types of bases, namely, "A" (Adenine), "G" (Guanine), "C" (Cytosine), and "T" (Thymine). The most easily oxidized guanine nucleotide (dG=deoxyguanosine), turns into oxidized 8-OHdG and is excreted in urine. It is said that such 8-OHdG (FIG. 16) is a biomarker that sharply reflects biological oxidation brought about by reactive oxygen. Liquid chromatography, and the ELISA method which uses monoclonal antibodies for specifically recognizing 8-OHdG, are given as methods for measuring the 8-OHdG. The present embodiment does not particularly restrict the method for measuring 8-OHdG.

The case in which this stress marker is measured shall be explained in the present embodiment.

First, the designated condition creation unit 114 creates information stating "The objective of this measurement is the widespread furnishing of vital signs and analysis results thereof, and there is a possibility that the subject's vital signs and analysis results thereof may be used by an indefinite number of persons", and information stating "Check the relationship between 8-OHdG in urine, and stress", and transmits such created information to the subject notification unit 107 in advance. In addition, the designated condition creation unit 114 creates information stating "Now recruiting subjects to participate in this measurement".

In this manner, a form is adopted in the present embodiment, which discloses the designated condition to an indefinite number of persons in advance, and obtains only the vital signs of persons accepting the designated condition. Furthermore, a form is adopted which distributes a measurement reagent needed for the measurement of 8-OHdG in urine only to the persons accepting the designated condition, and acquires vital signs measured by the vital sign measurement unit 102 which is a health toilet.

Next, the designated condition creation unit 114 creates a designated condition stating "Measure 8-OHdG and creatinine once a day, continually for seven days, using urine taken before going to sleep at night; answer questionnaire concerning stress daily", and transmits this to the subject notification unit 107. A structure is adopted in which the subject notification unit 107 which receives such notification displays such questionnaire, and the subject inputs the answer to such questionnaire using the additional information input unit 106.

Creatinine is a substance produced by muscles and it is said that the amount excreted in a day's urine is an almost fixed value depending on the amount of muscle a person has. Accordingly, when creatinine is used as a reference substance that is excreted in a fixed ratio, the effects of the dilution/concentration of urine can be compensated, and estimation of the "excretion amount in one day's urine" is made possible. An explanation regarding creatinine which is the reference material in this measurement of substance in urine can be given to the subject. Furthermore, it is also possible to adopt a structure in which creatinine is always measured when measurement of the vital sign is carried out using the health toilet, and is used in the normalization of the measured vital sign. In addition, the substance used in the normalization of the vital signs is not restricted to creatinine, and can be appropriately selected in accordance with the substance to be measured and the measurement method. The creatinine measurement methods are generally classified under the chemical measurement method (Jaffe method) and the enzymatic measurement method. The chemical measurement method (Jaffe method) makes use of the character of creatinine to react with picric acid under alkalinity and change its color to an orange-red color. The creatinine deaminase method and a creatinine amide hydrolase (creatinase) method exist in the enzymatic measurement method. The former, causes enzymes to act on the creatinine to form ammonia and measures this with the colorimetric method. The latter, changes creatinine into creatine using creatinase, and measures this creatine by the colorimetric method, using sarcosine oxydase and peroxidase. The present embodiment does not particulary restrict the measurement method.

The subject measures the 8-OHdG and creatinine in urine everyday before sleeping at night, using the vital sign measurement unit 102. The measuring-side transmission unit 104 transmits the following to the vital sign acquisition apparatus 110, via the communication network 130: the 8-OHdG and creatinine in urine measured using the vital sign measurement unit 102; the time of measurement, generated by the timing function of the measurement condition creation unit 103; and the questionnaire result inputted using the additional information input unit 106.

The compliance level creation unit 115 creates a compliance level on a per-measurement basis, based on whether or not the designated measurement item is measured, whether measurement is taken according to the designated condition, and the percentage of the questionnaire response ratio. The information analysis unit 112 analyzes the vital sign and accumulates this analysis information in the information accumulation unit 116.

The feedback information creation unit 117 creates point information based on the compliance level, and transmits this to the subject notification unit 107. The point information may be something that can be used for vital sign measurement such as measurement-related products and analysis information. In addition, the point information may also be used for something not related to vital sign measurement such as the miles in a mileage program, marketable securities, and weather forecasting services. The subject's performance of the measurement according to the designated condition leads to the obtainment of points.

According to the present embodiment, by giving a reward in return for furnishing information, subjects can be extensively recruited, and in addition, the subjects strive to measure vital signs more accurately. With this, effective and accurate information acquisition can be carried out. As the information accumulation unit 116 accumulates vital signs which are associated with the questionnaire responses, an effective database can be constructed. With this, factors having an effect on the vital signs, and information that is useful for analysis can be accumulated together with the vital signs, and as new findings concerning new measurement items can be extensively acquired, it is possible to construct a database that can further contribute to health management.

As described above, the purpose in the present embodiment is to check the relationship between the 8-OHdG in urine and stress, and construct a database that can contribute to health management. However, development of measurement reagents is also possible through the adoption the same configuration in which the measurement conditions are disclosed to the subject and measurement participants are recruited.

Moreover, in the case where there is a possibility that a subject's vital signs and analysis information thereof will be used by an indefinite number of persons other than the subject, as mentioned in the widespread acquisition of vital signs and construction of the database for providing information, it is necessary that obtainment of the subject's vital signs are carried out on the basis that such information usage is relayed to the subject in advance and that the subject's consent thereto has been obtained. Such notification of information usage to the subject can be carried out using the subject notification unit 107, and in addition, the act of consent thereto can be carried out using the personal information input unit 105. Furthermore, the database can be managed on a per-vital sign measurement apparatus basis. In addition, through a registration with personal identification information, the database can also be managed on a per-person basis.

Although only some exemplary embodiments of this invention have been described in detail above, those skilled in the art will readily appreciate that many modifications are possible in the exemplary embodiments without materially departing from the novel teachings and advantages of this invention. Accordingly, all such modifications are intended to be included within the scope of this invention.

## Claims

1. A vital sign processing apparatus comprising:
a vital sign measurement unit operable to measure a vital sign of a subject;
a designated condition notification unit operable to notify the subject of a designated condition for measuring the vital sign using said vital sign measurement unit;
a measurement condition recognition unit operable to recognize, without input from the subject, a measurement condition at a time the vital sign is measured by the subject using said vital sign measurement unit;
a condition comparison unit operable to compare the designated condition notified by said designated condition notification unit with the measurement condition recognized by said measurement condition recognition unit; and
a presentation unit operable to present a result of the comparison made by said condition comparison unit.

2. The vital sign processing apparatus according to Claim 1, further comprising
a compliance level creation unit operable to create a compliance level for the designated condition, based on the result of the comparison made by said condition comparison unit,
wherein said presentation unit is operable to present the compliance level created by said compliance level creation unit.

3. The vital sign processing apparatus according to Claim 2, further comprising
an additional information input unit through which additional information is inputted, the additional information being information that cannot be recognized by said measurement condition recognition unit,
wherein said condition comparison unit is operable to compare the designated condition with the additional information.

4. The vital sign processing apparatus according to Claim 3, further comprising:
an information accumulation unit operable to store the vital sign measured using said vital sign measurement unit, the measurement condition created by said measurement condition recognition unit, and the additional information inputted through said additional information input unit, in association with each other; and
an accumulation judgment unit operable to judge, based on the compliance level, whether or not to accumulate the vital sign, the measurement condition, and the additional information, in said information accumulation unit.

5. The vital sign processing apparatus according to Claim 2, further comprising
a re-measurement information creation unit operable to judge whether or not re-measuring is necessary, based on the compliance level,
wherein, in the case where said re-measurement information creation unit judges that re-measuring is necessary, and creates re-measurement information, said presentation unit is operable to present the re-measurement information.

6. The vital sign processing apparatus according to Claim 2, further comprising
a feedback information creation unit operable to create, based on the compliance level, feedback information for increasing motivation of the subject to take a measurement according to the designated condition,
wherein said presentation unit is operable to present the feedback information.

7. A vital sign processing system comprising a vital sign measurement apparatus that obtains a vital sign from a subject, and a vital sign acquisition apparatus that acquires the vital sign from said vital sign measurement apparatus,
wherein said vital sign acquisition apparatus includes:
a designated condition creation unit operable to create a designated condition for measuring the vital sign using said vital sign measurement apparatus;
an acquisition apparatus-side output unit operable to output the designated condition, and
said vital sign measurement apparatus includes:
a measurement apparatus-side input unit operable to receive the designated condition;
a designated condition notification unit operable to notify the subject of the designated condition created by said designated condition creation unit;
a vital sign measurement unit operable to measure the vital sign of the subject;
a measurement condition recognition unit operable to recognize, without input from the subject, a measurement condition at a time the vital sign is measured by the subject using said vital sign measurement unit; and
a measurement apparatus-side output unit operable to output the vital sign and the measurement condition,
said vital sign acquisition apparatus further includes:
an acquisition apparatus-side input unit operable to receive the vital sign and the measurement condition;
a condition comparison unit operable to compare the designated condition with the measurement condition, and
said acquisition apparatus-side output unit is operable to output a result of the comparison made by said condition comparison unit.

8. The vital sign processing system according to Claim 7,
wherein said vital sign acquisition apparatus further includes
a compliance level creation unit operable to create a compliance level for the designated condition, based on the result of the comparison made by said condition comparison unit, and
said acquisition apparatus-side output unit is operable to output the compliance level created by the compliance level creation unit.

9. The vital sign processing system according to Claim 7,
wherein said vital sign acquisition apparatus further includes:
an information accumulation unit operable to store the vital sign and the measurement condition in association with each other; and
an accumulation judgment unit operable to judge, based on the compliance level, whether or not to accumulate the vital sign and the measurement condition, in said information accumulation unit.

10. The vital sign processing system according to Claim 7,
wherein said vital sign measurement apparatus further includes
an additional information input unit through which additional information is inputted, the additional information being information that cannot be recognized by said measurement condition recognition unit,
said measurement apparatus-side output unit is operable to further output the additional information,
said acquisition apparatus-side input unit is operable to further receive the additional information, and
said condition comparison unit is operable to further compare the designated condition with the additional information.

11. The vital sign processing system according to Claim 7,
wherein said vital sign acquisition apparatus further includes
a feedback information creation unit operable to create, based on the compliance level, feedback information for increasing motivation of the subject to take a measurement according to the designated condition, and
said acquisition apparatus-side output unit is operable to output the feedback information.

12. A vital sign acquisition apparatus that acquires a vital sign from a vital sign measurement apparatus that obtains the vital sign from a subject, said vital sign acquisition apparatus comprising:
a designated condition creation unit operable to create a designated condition for measuring the vital sign using said vital sign measurement apparatus;
an output unit operable to output the designated condition;
an input unit operable to obtain, from said vital sign measurement apparatus, the vital sign measured by the subject using said vital sign measurement apparatus, and a measurement condition at a time the vital sign is measured; and
a condition comparison unit operable to compare the designated condition and the measurement condition,
wherein said output unit is operable to output a result of the comparison made by said condition comparison unit.

13. The vital sign acquisition apparatus according to Claim 12, further comprising
a compliance level creation unit operable to create a compliance level for the designated condition, based on the result of the comparison made by said condition comparison unit,
wherein said output unit is operable to output the compliance level created by said compliance level creation unit.

14. The vital sign acquisition apparatus according to Claim 12, further comprising:
an information accumulation unit operable to store the vital sign and the measurement condition in association with each other; and
an accumulation judgment unit operable to judge, based on the compliance level, whether or not to accumulate the vital sign and the measurement condition, in said information accumulation unit.

15. A vital sign processing method comprising the steps of:
notifying a subject of a designated condition for measurement of a vital sign by the subject;
measuring the vital sign of the subject;
recognizing, without input from the subject, a measurement condition at a time of said measuring of the vital sign;
comparing the designated condition with the measurement condition; and
presenting a result of said comparison.

16. A program that causes a computer to execute the steps included in the vital sign processing method in Claim 15.
